# EUROPEAN PATENT SPECIFICATION

(11) **EP 4 311 587 B1**
(45) Date of publication and mention of the grant of the patent: **24.09.2025**
(21) Application number: 23188155.8
(22) Date of filing: 27.07.2023
(51) Int. Cl.: A61L 2/04, B01D 29/05, B01D 35/027

(54) **FILTER FOR A MACHINE FOR TREATING OBJECTS AND RELATED MACHINE**
FILTER FÜR EINE MASCHINE ZUR BEHANDLUNG VON OBJEKTEN UND ZUGEHÖRIGE MASCHINE
FILTRE POUR MACHINE DE TRAITEMENT D'OBJETS ET MACHINE ASSOCIÉE

(30) Priority: 27.07.2022 IT 202200013984
(43) Date of publication of application: 31.01.2024
(73) Proprietor: Steelco S.p.A., 31039 Riese Pio X (IT)
(72) Inventor: Belluco, Nicola, Tribano (PD) (IT)
(74) Representative: Petraz, Gilberto Luigi

(56) References cited:
- WO-A1-01/51166
- US-A- 5 143 604
- US-A1- 2021 339 296

## Description

### SCOPE OF APPLICATION

The present invention concerns a filter for a machine for treating objects and the related machine for treating objects, which find application, for example but without limitation to the generality, in the field of sanitization, disinfection, thermodisinfection and/or sterilization of surgical instruments or for hospital use, or of laboratory devices, instruments or glassware.

### BACKGROUND OF THE INVENTION

Machines for treating objects are known, such as surgical instruments or for hospital use, or of laboratory devices, instruments or glassware, which comprise a treatment chamber having a bottom wall for the collection of washing water.

Normally the bottom wall is shaped to convey the washing water toward a collection tank positioned below the treatment chamber, where it is collected and made available for recirculation.

The collection tank has an upper aperture, communicating with the treatment chamber, in correspondence with which a filter is positioned which is suitable for preventing any particles or corpuscles of dirt or contaminants, for example particles of organic and/or inorganic material coming from the operating rooms or laboratories, from reaching the inside of the collection tank so that they are not recirculated.

Normally, a filter of known type comprises a filtering panel having the function of allowing the passage of the washing water toward the collection tank and of blocking the passage of the dirt particles. This filtering panel is fixed to the perimeter of the upper aperture of the collection tank by means of screws or other similar devices.

This solution, although widely used, has some drawbacks.

A first drawback consists in the fact that the screws for fixing the filtering panel can deform the edge of the latter to such an extent that it does not perfectly adhere to the perimeter of the upper aperture defining, in an undesired way, also apertures or passages through which dirt particles can pass together with the washing water.

Another drawback consists in the fact that the screws for fixing the filtering panel make it slow and difficult to assemble and disassemble it and slow down the maintenance operations of the machine for treating objects.

A support assembly of a filter is described for example in document US5143604A.

Document WO01/51166A discloses a device, method and system for removing contaminants from a liquid.

Document US2021/339296A discloses an electrical sanitizing device.

There is therefore the need to perfect a filter for a machine for treating objects that can overcome at least one of the disadvantages of the state of the art.

To do this, it is necessary to solve the technical problem of uniformly letting the filtering panel adhere to the perimeter of the upper aperture of the collection tank.

In particular, one purpose of the present invention is to realize a filter for a machine for treating objects that allows to eliminate, or at least minimize, the amount of dirt particles that reach the collection tank.

Another purpose of the present invention is to realize a filter for a machine for treating objects that is easy to assemble and economical to produce.

A further purpose of the present invention is to realize a filter for a machine for treating objects that is easy and quick to assemble and disassemble on the collection tank.

The Applicant has devised, tested and embodied the present invention to overcome the shortcomings of the state of the art and to obtain these and other purposes and advantages.

### SUMMARY OF THE INVENTION

The present invention is set forth and characterized in the independent claim/s. The dependent claims describe other characteristics of the present invention or variants to the main inventive idea.

In accordance with the above purposes, and to resolve the technical problem disclosed above in a new and original way, also achieving considerable advantages compared to the state of the prior, a filter according to the present invention comprises a filtering member having a central body defining an external surface and an internal surface opposite to said external surface and an edge connected to said central body. Said central body is concave in shape and has a bottom wall.

In accordance with one aspect of the present invention, the filter also comprises a retention member configured to selectively flex said filtering member and take said edge toward a direction substantially normal, or perpendicular, to said internal surface, away from said external surface. Said retention member comprises a central portion and lateral portions inserted into a corresponding treatment portion defined by said edge. Said central portion is provided with a central section configured to be placed under the bottom wall of said central body and with at least two oblique lateral sections that develop on the sides of said central section and connect it to said lateral portions of said retention member. Said central portion is connected to said central body by a selectively adjustable connection member and configured to induce in said retention member a bending moment adapted to take said edge toward said normal, or perpendicular, direction to said internal surface.

Said retention member assumes a substantially "M" shape that increases its elastic properties and the ability to flex the filtering member.

According to another aspect of the invention, said lateral portions are at a higher elevation than said central section.

According to another aspect of the invention, said oblique sections develop on diametrically opposite sides of said central section.

According to another aspect of the invention, said oblique sections develop specularly with respect to said central section.

In accordance with another aspect of the present invention, said retention member comprises interlocking portions connected to said lateral portions, which develop transversely with respect to the latter and which are configured to be inserted into a collection tank of said machine.

In accordance with another aspect of the present invention, said interlocking portions are configured to flex toward said central portion consequently taking said edge toward said direction normal, or perpendicular, to said internal surface.

In accordance with another aspect of the present invention, said retention member comprises at least one oblong metal plate bent in order to define in a single body said central portion, said at least one lateral portion and said at least one interlocking portion.

In accordance with another aspect of the present invention, the filter comprises two lateral portions each disposed in correspondence with an end of said lateral sections of said central portion and two interlocking portions each of which is disposed in correspondence with a respective lateral portion.

In accordance with another aspect of the present invention, each interlocking portion comprises, in correspondence with its free end, a convergent portion facing toward the inside of the retention member and suitable to facilitate the insertion of the retention member into a collection tank of said machine.

In accordance with another aspect of the present invention, said connection member comprises at least one screw passing through said central portion and said central body, and a contrast element on which said screw is screwed, wherein said central body and said central portion are interposed between the head of said screw and said contrast element.

In accordance with a further aspect of the present invention, a machine for treating objects comprises a treatment chamber and a collection tank underneath said treatment chamber and communicating with it by means of an aperture.

In accordance with one aspect of the present invention, the machine further comprises a filter in accordance with the present invention, wherein said central body is overlapping on said aperture, said internal surface is facing toward the inside of said collection tank, said edge is positioned substantially in correspondence with the perimeter of said aperture, and said filtering member is flexed by said retention member in order to press at least a portion of said edge against the perimeter of said upper aperture.

### DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a three-dimensional exploded view of a portion of a machine for treating objects comprising a filter in accordance with an embodiment of the present invention;
- fig. 2 is an axonometric view of a filter in accordance with an embodiment of the present invention;
- fig. 3 is a sectional view of a part of the filter of fig. 2;
- fig. 4 is a sectional view of the filter of fig. 2 when it is not inserted in the machine for treating objects;
- fig. 5 is a sectional view of the filter of fig. 2 when it is inserted into the object processing machine.

We must clarify that in the present description the phraseology and terminology used, as well as the figures in the attached drawings also as described, have the sole function of better illustrating and explaining the present invention, their function being to provide a non-limiting example of the invention itself, since the scope of protection is defined by the claims.

To facilitate comprehension, the same reference numbers have been used, where possible, to identify identical common elements in the drawings. It is understood that elements and characteristics of one embodiment can be conveniently combined or incorporated into other embodiments without further clarifications.

### DESCRIPTION OF EMBODIMENTS

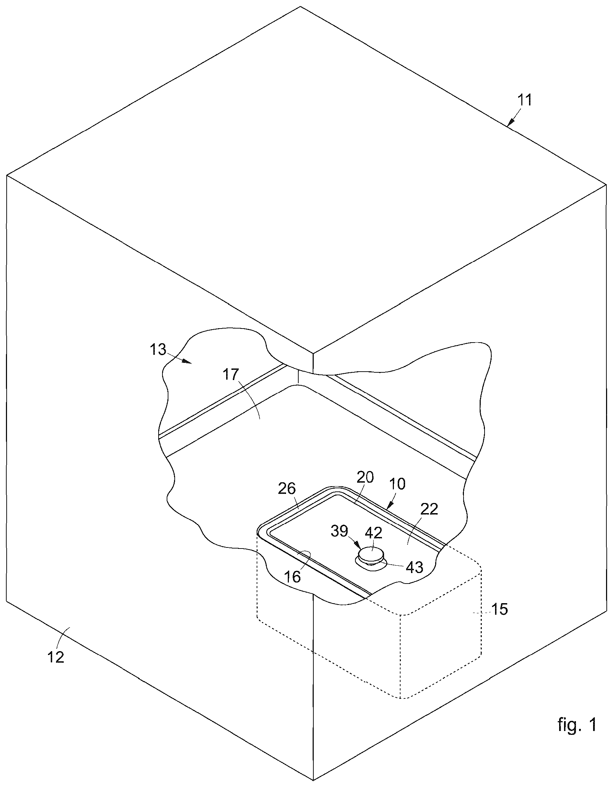

Figure 1 is used to describe embodiments of a filter 10 configured to be inserted in a machine for treating objects 11, hereinafter machine 11, which may be, by way of non-limiting example, a machine for sanitizing, disinfecting, thermodisinfecting and/or sterilizing surgical instruments or for hospital use, or laboratory devices, instruments or glassware.

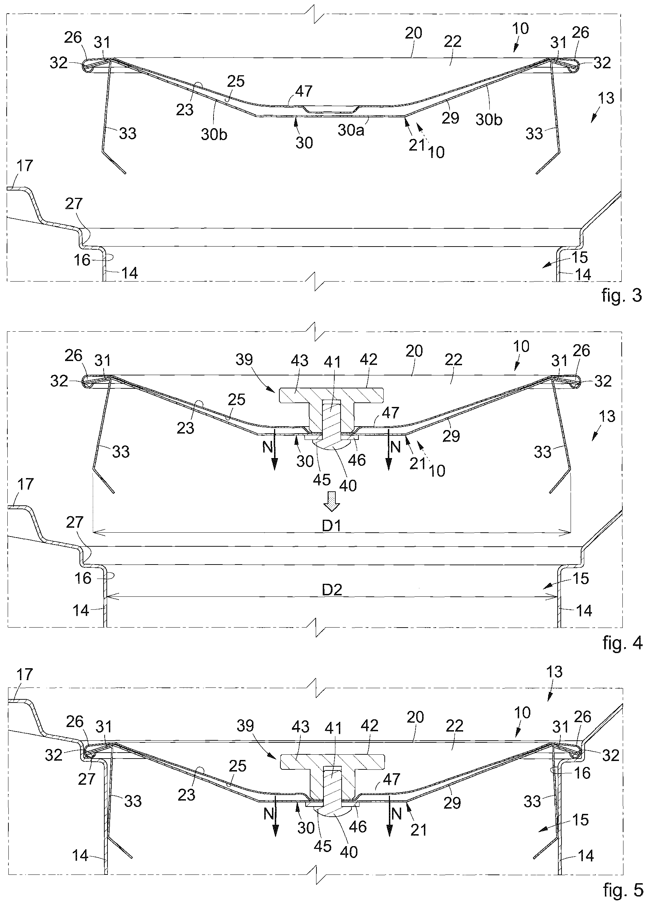

In particular, the machine 11 comprises a frame 12 defining a treatment chamber 13, and a collection tank 15 (figs. 3, 4 and 5) disposed below the treatment chamber 13 and having an upper aperture 16. The treatment chamber 13 has a bottom wall 17 shaped so as to convey the washing water toward the upper aperture 16 of the collection chamber 15.

The machine 11 also comprises washing members (not shown in the drawings), of known type and which will not be described in detail, configured to withdraw the washing water from the collection tank 15 and recirculate it inside the treatment chamber 13.

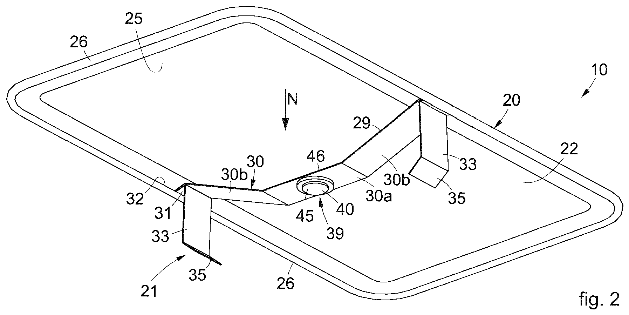

The filter 10 is disposed in correspondence with the upper aperture 16 of the collection tank 15 and comprises a filtering member 20 and a retention member 21 (fig. 2) connected to the filtering member 20 and configured to retain the filter 10 in position with respect to the machine 11, and in particular with respect to the upper aperture 16.

The filtering member 20 has a central body 22 having a plan shape conjugated with the section of the upper aperture 16 and is configured to allow the passage of the washing water toward the collection tank 15 and to block the passage of corpuscles or particles of dirt or contaminants present in the washing water so that they do not reach the inside of the collection chamber 15.

The central body 22 defines an external surface 23 which, in use, is facing toward the outside of the collection tank 15 and an internal surface 25 which, in use, is facing toward the inside of the collection tank 15.

The filtering member 20 comprises an edge 26, in particular of annular shape, connected to the central body 22 and configured to stabilize the position of the filtering member 20 with respect to the upper aperture 16. Said filtering member 20 comprises a bottom wall 47, positioned substantially centrally and at a lower elevation than said edge 26.

In this case, in correspondence with the perimeter of the upper aperture 16 there is a substantially flat seat 27 and the edge 26 is configured to overlap the seat 27.

The retention member 21 is configured to flex the filtering member 20 and press said edge 26 toward a normal direction N to the internal surface 25, or substantially perpendicular to the latter, pressing it toward the inside of the collection tank 15, i.e. away from the external surface 23.

The retention member 21 is connected to the filtering member 20 and defines a central portion 30 having a shape substantially conjugated with the shape of the central body 22 of the filtering member 20 and two lateral portions 31.

Each lateral portion 31 is inserted and retained by mechanical interference in a corresponding retention portion 32 defined by the edge 26. In particular, the treatment portion 32 is annular and develops over the entire edge 26. Said edge 26 and said treatment portion 32 have in this case a substantially rectangular shape.

In the example provided herein, see figures 4 and 5, in correspondence with the retention portion 32 the edge 26 is folded on itself and its section has the shape of a "C" into which the lateral portion 31 is inserted.

The retention member 21 also defines two interlocking portions 33, each of which develops from a corresponding lateral portion 31 and is configured to be inserted inside the collection tank 15 and to cooperate, by friction, with the walls 14 thereof.

The two interlocking portions 33 are divergent from each other and each of them comprises, in correspondence with its free end, a convergent portion 35 suitable to facilitate the insertion of the retention member 21 inside the collection tank 15. In other words, the convergent portions 35 act as a lead-in for the insertion of the retention member 21 inside the collection tank 15.

The central body 22 of the filtering member 20, as is clear from the figures, has a concave shape, i.e. like a basin or tank, facing upwards. Said central portion 30 of the retention element 21 has a substantially shape conjugated with that of said central body 22 and is consequently concave.

In particular, see fig. 2, said central portion 30 comprises a central section 30a configured to be placed under the bottom wall 47 of said central body 22. Said central section 30a is substantially horizontal. At the sides of said central section 30a there are provided two oblique lateral sections 30b connecting the central section 30a with said lateral portions 31. Said lateral sections 30b develop on diametrically opposite sides of said central section 30a.

Said central section 30a is located at a lower elevation than said lateral portions 31 which are connected to it by said lateral sections 30b. Said lateral portions 31 are at a higher elevation than said central section 30a. Said lateral sections 30b are directed from the bottom upwards to the sides of said central section 30a, therefore they are substantially directed from the bottom wall 47 to the side edge 26 of the central body 22 of the filtering member 20.

Said lateral sections 30b are positioned specularly to the sides of said central section 30a, so that the retention element 21 flexes similarly on both sides, i.e. in particular in correspondence with the side walls 31 and the interlocking portions 33.

The retention member 21, as can be noted for example in fig. 2 and fig. 5, assumes a substantially "M" shape, which increases its elastic properties and the ability to flex the filtering member 20, in order to press the edge 26 toward a normal direction N to said internal surface 25.

Preferably, the retention member 21 consists essentially of a metal plate 29, preferably made of harmonic steel, bent to define, in a single body, the central portion 30, the lateral portions 31 and the interlocking portions 33.

In particular, in correspondence with each end of the central portion 30 the metal plate 29 is folded on itself and defines one of the two lateral portions 31. Furthermore, in correspondence with the lateral portions 31, the metal plate 29 develops in a substantially transverse direction with respect to the central portion 30 thus defining the two interlocking portions 33.

However, the person skilled in the art easily understands that in other possible embodiments the retention member 21 can be constituted by a plurality of metal plates connected to each other, for example by welding.

The metal plate 29 is connected to the central body 22 of the filtering member 20 by means of a connection member 39 selectively adjustable to allow a user to select the level of force with which to press the metal plate 29 against the central body 22.

In particular, the connection member 39 comprises a screw 40 having the shank 41 passing through the central portion 30 of the metal plate 29 and through the central body 22 of the filtering member 20, in particular through a through hole obtained in the bottom wall 47 of said central body 22.

The connection member 39 also comprises a contrast element 42 into which the shank 41 of the screw 40 is screwed and further comprises a gripping portion 43 to allow a user to adjust the force with which the screw 40 is screwed to the contrast element 42.

In this case, the central body 22 and the metal plate 29 are interposed between the contrast element 42 and the head 45 of the screw 40. Optionally, a washer 46 may be disposed between the head 45 of the screw 40 and the metal plate 29.

The metal plate 29 is configured to press the edge 26 of the filtering member 20 in a normal direction N to the internal surface 25, or substantially perpendicular to the latter, so that, in use, the edge 26 is pressed against the perimeter of the upper aperture 16. This allows to eliminate, or at least reduce, leaks of dirt particles between the edge 26 and the perimeter of the upper aperture 16. In this way the washing water recirculated from the collection tank 15 toward the treatment chamber 13 is always free of dirt particles.

In particular, the connection member 39 is configured to induce a bending moment in the metal plate 29 which takes the lateral portions 31 to press against the edge 26 of the filtering member 20.

By way of example only, see figure 3 in which the metal plate 29 is disconnected from the filter 17 and figure 4 in which the metal plate 29 is connected to the filter 17 by means of the connection member 39. In the operating condition represented by way of example in figure 4, the metal plate 29 is bent and deformed by the action of the connection member 39, with respect to the rest condition represented by way of example in figure 3.

The amount of force with which the lateral portions 31 press against the respective portion of the edge 26 of the filtering member 20 depends on the closing torque with which the screw 40 is screwed to the contrast element 42. The greater the closing torque the greater the force that the lateral portions 31 apply to the edge 26 of the filtering member 20 in order to press it against the perimeter of the upper aperture 16.

Furthermore, optionally, the greater distance D1 between the two interlocking portions 33, when they are outside the collection tank 15 (fig. 4), is greater than the distance D2 between the walls 14 of the collection tank 15 with which they cooperate.

Such a conformation is advantageous in that, when the two interlocking portions 33 are inside the collection tank 15 and cooperate with the walls 14 (fig. 5), they flex each other toward each other. This increases the bending moment applied to the metal plate 29 and thus also the force that the lateral portions 31 apply to the edge 26 of the filtering member 20 in order to press it against the perimeter of the upper aperture 16.

It is clear that modifications and/or additions of parts may be made to the filter 10 as described heretofore, without departing from the field and scope of the present invention, as defined by the claims.

It is also clear that, although the present invention has been described with reference to some specific examples, a person of skill in the art shall certainly be able to achieve other equivalent forms of filter having the characteristics as set forth in the claims and hence all coming within the field of protection defined thereby.

In the following claims, the sole purpose of the references in brackets is to facilitate their reading and they must not be considered as restrictive factors with regard to the field of protection defined by the claims.

## Claims

1. Filter (10) for a machine (11) for treating objects, comprising a filtering member (20) having a central body (22) of concave shape, provided with a bottom wall (47) and defining an external surface (23) and an internal surface (25) opposite each other and an edge (26) connected to said central body (22), **characterized in that** it comprises a retention member (21) configured to selectively flex said filtering member (20) and take said edge (26) toward a normal direction (N), substantially perpendicular to said internal surface (25), wherein said retention member (21) comprises a central portion (30) and lateral portions (31) inserted into a corresponding treatment portion (32) defined by said edge (26), wherein said central portion (30) is provided with a central section (30a) configured to be placed below the bottom wall (47) of said central body (22) and with at least two oblique lateral sections (30b) which develop on the sides of said central section (30a) and connect it to said lateral portions (31) of said retention member (21), and wherein said central portion (30) of the retention member (21) is connected to said central body (22) by a selectively adjustable connection member (39) configured to induce therein a bending moment adapted to take said edge (26) of the filtering member (20) toward a normal direction (N) substantially perpendicular to said internal surface (25) of the central body (22).

2. Filter (10) as in claim 1, **characterized in that** said lateral portions (31) are at a higher elevation than said central section (30a).

3. Filter (10) as in claim 1 or 2, **characterized in that** said oblique sections (30b) develop on diametrically opposite sides of said central section (30a).

4. Filter (10) as in claim 3, **characterized in that** said oblique sections (30b) develop specularly with respect to said central section (30a).

5. Filter (10) as in any claim hereinbefore, **characterized in that** said retention member (21) comprises interlocking portions (33) connected to said lateral portions (31), which develop transversely with respect to the latter and which are configured to be inserted into a collection tank (15) of said machine (11).

6. Filter (10) as in claim 5, **characterized in that** said interlocking portions (33) are configured to flex toward said central portion (30) consequently taking said edge (26) toward a normal direction (N) to said internal surface (25).

7. Filter (10) as in any claim hereinbefore, **characterized in that** said retention member (21) comprises at least one oblong metal plate (29) bent in order to define in a single body said central portion (30), said at least one lateral portion (31) and said at least one interlocking portion (33).

8. Filter (10) as in any claim hereinbefore, **characterized in that** it comprises two lateral portions (31) each disposed in correspondence with an end of said lateral sections (30b) of said central portion (30) and two interlocking portions (33) each of which is disposed in correspondence with a respective lateral portion (31).

9. Filter (10) as in claim from 3 to 6, **characterized in that** each interlocking portion (33) comprises, in correspondence with its free end, a convergent portion (35) facing toward the inside of the retention member (21) and suitable to facilitate the insertion of the retention member (21) inside the collection tank (15).

10. Filter (10) as in any claim hereinbefore, **characterized in that** said connection member (39) comprises at least one screw (41) passing through said central portion (30) and said central body (22), and a contrast element (42) on which said screw (41) is screwed, wherein said central body (22) and said central portion (30) are interposed between the head (45) of said screw (41) and said contrast element (42).

11. Machine (11) for treating objects comprising a treatment chamber (13) and a collection tank (15) underneath said treatment chamber (13) and communicating with it by means of an aperture (16), **characterized in that** said machine (11) further comprises a filter (10) as in any claim from 1 to 10, in which said central body (22) is overlapping on said aperture (16), said internal surface (25) is facing toward the inside of said collection tank (15), said edge (26) is positioned substantially in correspondence with the perimeter of said aperture (16), and said filtering member (20) is flexed by said retention member (21) in order to press at least a portion of said edge (26) against the perimeter of said upper aperture (16).

## Patentansprüche

1. Filter (10) für eine Maschine (11) zur Behandlung von Gegenständen, umfassend ein Filterelement (20) mit einem zentralen Körper (22) von konkaver Form, der mit einer Bodenwand (47) versehen ist und der eine Außenfläche (23) und eine dazu gegenüberliegende Innenfläche (25) sowie eine mit dem zentralen Körper (22) verbundene Kante (26) definiert, **dadurch gekennzeichnet, dass** er ein Halteelement (21) umfasst, das so konfiguriert ist, dass es das Filterelement (20) selektiv biegt und es die Kante (26) in eine Normalrichtung (N), im Wesentlichen senkrecht zur Innenfläche (25), führt, wobei das Halteelement (21) einen zentralen Abschnitt (30) und seitliche Abschnitte (31) umfasst, die in einen entsprechenden, durch die Kante (26) definierten Behandlungsabschnitt (32) eingesetzt sind, wobei der zentrale Abschnitt (30) mit einem zentralen Abschnitt (30a) versehen ist, der so konfiguriert ist, dass er unterhalb der Bodenwand (47) des zentralen Körpers (22) platziert ist und mit mindestens zwei schrägen seitlichen Abschnitten (30b), die sich an den Seiten des zentralen Abschnitts (30a) erstrecken und es mit den seitlichen Abschnitten (31) des Halteelements (21) verbinden, versehen ist, und wobei der zentrale Abschnitt (30) des Halteelements (21) mit dem zentralen Körper (22) durch ein wahlweise einstellbares Verbindungselement (39) verbunden ist, das so konfiguriert ist, dass darin ein Biegemoment erzeugt wird, das die Kante (26) des Filterelements (20) in eine Normalrichtung (N), im Wesentlichen senkrecht zur Innenfläche (25) des zentralen Körpers (22), drückt.

2. Filter (10) nach Anspruch 1, **dadurch gekennzeichnet, dass** die seitlichen Abschnitte (31) höher liegen als der zentrale Abschnitt (30a).

3. Filter (10) nach Anspruch 1 oder 2, **dadurch gekennzeichnet, dass** sich die schrägen Abschnitte (30b) auf diametral gegenüberliegenden Seiten des zentralen Abschnitts (30a) erstrecken.

4. Filter (10) nach Anspruch 3, **dadurch gekennzeichnet, dass** sich die schrägen Abschnitte (30b) in Bezug auf den zentralen Abschnitt (30a) spiegelbildlich erstrecken.

5. Filter (10) nach einem der vorhergehenden Ansprüche, **dadurch gekennzeichnet, dass** das Halteelement (21) Verriegelungsabschnitte (33) aufweist, die mit den seitlichen Abschnitten (31) verbunden sind, sich quer zu diesen erstrecken und zum Einsetzen in einen Sammelbehälter (15) der Maschine (11) konfiguriert sind.

6. Filter (10) nach Anspruch 5, **dadurch gekennzeichnet, dass** die Verriegelungsabschnitte (33) so konfiguriert sind, dass sie sich in Richtung des zentralen Abschnitts (30) biegen, wodurch die Kante (26) in eine Normalrichtung (N) zur Innenfläche (25) bewegt wird.

7. Filter (10) nach einem der vorhergehenden Ansprüche, **dadurch gekennzeichnet, dass** das Halteelement (21) mindestens eine längliche Metallplatte (29) umfasst, die gebogen ist, um in einem einzigen Körper den zentralen Abschnitt (30), den mindestens einen Seitenabschnitt (31) und den mindestens einen Verriegelungsabschnitt (33) zu definieren.

8. Filter (10) nach einem der vorhergehenden Ansprüche, **dadurch gekennzeichnet, dass** er zwei seitliche Abschnitte (31), die jeweils in Übereinstimmung mit einem Ende der seitlichen Abschnitte (30b) des zentralen Abschnitts (30) angeordnet sind, sowie zwei Verriegelungsabschnitte (33), die jeweils in Übereinstimmung mit einem jeweiligen seitlichen Abschnitt (31) angeordnet sind, umfasst.

9. Filter (10) nach einem der Ansprüche 3 bis 6, **dadurch gekennzeichnet, dass** jeder Verriegelungsabschnitt (33) an seinem freien Ende einen konvergenten Abschnitt (35) aufweist, der zur Innenseite des Halteelements (21) zeigt und dazu geeignet ist, das Einsetzen des Halteelements (21) in den Sammelbehälter (15) zu erleichtern.

10. Filter (10) nach einem der vorhergehenden Ansprüche, **dadurch gekennzeichnet, dass** das Verbindungselement (39) mindestens eine Schraube (41) umfasst, die durch den zentralen Abschnitt (30) und den zentralen Körper (22) verläuft, sowie ein Kontrastelement (42), auf das die Schraube (41) geschraubt ist, wobei der zentrale Körper (22) und der zentrale Abschnitt (30) zwischen dem Kopf (45) der Schraube (41) und dem Kontrastelement (42) angeordnet sind.

11. Maschine (11) zum Behandeln von Objekten, umfassend eine Behandlungskammer (13) und einen Sammeltank (15) unterhalb der Behandlungskammer (13), der mit dieser über eine Öffnung (16) in Verbindung steht, **dadurch gekennzeichnet, dass** die Maschine (11) außerdem einen Filter (10) gemäß einem der Ansprüche 1 bis 10 umfasst, bei dem der zentrale Körper (22) die Öffnung (16) überlappt, die Innenfläche (25) in Richtung des Inneren des Sammeltanks (15) zeigt, die Kante (26) im Wesentlichen in Übereinstimmung mit dem Umfang der Öffnung (16) positioniert ist und das Filterelement (20) durch das Halteelement (21) gebogen ist, um zumindest einen Abschnitt der Kante (26) gegen den Umfang der oberen Öffnung (16) zu drücken.

## Revendications

1. Filtre (10) pour une machine (11) de traitement d'objets, comprenant un élément filtrant (20) présentant un corps central (22) de forme concave, pourvu d'une paroi inférieure (47) et définissant une surface externe (23) et une surface interne (25) opposées l'une à l'autre et un bord (26) relié audit corps central (22), **caractérisé en ce qu'**il comprend un élément de retenue (21) configuré pour faire fléchir sélectivement ledit élément filtrant (20) et amener ledit bord (26) vers une direction normale (N), sensiblement perpendiculaire à ladite surface interne (25), dans lequel ledit élément de retenue (21) comprend une partie centrale (30) et des parties latérales (31) insérées dans une partie de traitement correspondante (32) définie par ledit bord (26), dans lequel ladite partie centrale (30) est pourvue d'une section centrale (30a) configurée pour être placée sous la paroi inférieure (47) dudit corps central (22) et d'au moins deux sections latérales obliques (30b) qui se développent sur les côtés de ladite section centrale (30a) et la relient auxdites parties latérales (31) dudit élément de retenue (21), et dans lequel ladite partie centrale (30) de l'élément de retenue (21) est reliée audit corps central (22) par un élément de liaison ajustable de manière sélective (39) configuré pour y induire un moment de flexion adapté pour amener ledit bord (26) de l'élément de filtrage (20) vers une direction normale (N) sensiblement perpendiculaire à ladite surface interne (25) du corps central (22).

2. Filtre (10) selon la revendication 1, **caractérisé en ce que** lesdites parties latérales (31) sont à une élévation plus élevée que ladite section centrale (30a).

3. Filtre (10) selon la revendication 1 ou 2, **caractérisé en ce que** lesdites sections obliques (30b) se développent sur des côtés diamétralement opposés de ladite section centrale (30a).

4. Filtre (10) selon la revendication 3, **caractérisé en ce que** lesdites sections obliques (30b) se développent de manière spéculaire par rapport à ladite section centrale (30a).

5. Filtre (10) selon l'une quelconque des revendications précédentes, **caractérisé en ce que** ledit élément de retenue (21) comprend des parties de verrouillage mutuel (33) reliées auxdites parties latérales (31), qui se développent transversalement par rapport à ces dernières et qui sont configurées pour être insérées dans un réservoir de collecte (15) de ladite machine (11).

6. Filtre (10) selon la revendication 5, **caractérisé en ce que** lesdites parties de verrouillage mutuel (33) sont configurées pour fléchir vers ladite partie centrale (30) en conséquence en amenant ledit bord (26) vers une direction normale (N) par rapport à ladite surface interne (25).

7. Filtre (10) selon l'une quelconque des revendications précédentes, **caractérisé en ce que** ledit élément de retenue (21) comprend au moins une plaque métallique oblongue (29) pliée afin de définir dans un corps unique ladite partie centrale (30), ladite au moins une partie latérale (31) et ladite au moins une partie de verrouillage mutuel (33).

8. Filtre (10) selon l'une quelconque des revendications précédentes, **caractérisé en ce qu'**il comprend deux parties latérales (31) disposées chacune en correspondance avec une extrémité desdites sections latérales (30b) de ladite partie centrale (30) et deux parties de verrouillage mutuel (33) dont chacune est disposée en correspondance avec une partie latérale respective (31).

9. Filtre (10) selon les revendications 3 à 6, **caractérisé en ce que** chaque partie de verrouillage mutuel (33) comprend, en correspondance avec son extrémité libre, une partie convergente (35) orientée vers l'intérieur de l'élément de retenue (21) et appropriée pour faciliter l'insertion de l'élément de retenue (21) à l'intérieur du réservoir de collecte (15).

10. Filtre (10) selon l'une quelconque des revendications précédentes, **caractérisé en ce que** ledit élément de liaison (39) comprend au moins une vis (41) passant à travers ladite partie centrale (30) et ledit corps central (22), et un élément complémentaire (42) sur lequel ladite vis (41) est vissée, dans lequel ledit corps central (22) et ladite partie centrale (30) sont interposés entre la tête (45) de ladite vis (41) et ledit élément complémentaire (42).

11. Machine (11) pour traiter des objets comprenant une chambre de traitement (13) et un réservoir de collecte (15) sous ladite chambre de traitement (13) et communiquant avec celle-ci par l'intermédiaire d'une ouverture (16), **caractérisée en ce que** ladite machine (11) comprend en outre un filtre (10) selon l'une quelconque des revendications 1 à 10, dans lequel ledit corps central (22) chevauche ladite ouverture (16), ladite surface interne (25) est orientée vers l'intérieur dudit réservoir de collecte (15), ledit bord (26) est positionné sensiblement en correspondance avec le périmètre de ladite ouverture (16), et ledit élément filtrant(20) est fléchi par ledit élément de retenue (21) afin de presser au moins une partie dudit bord (26) contre le périmètre de ladite ouverture supérieure (16).
